# EUROPEAN PATENT SPECIFICATION

(11) **EP 3 687 969 B1**
(45) Date of publication and mention of the grant of the patent: **02.08.2023**
(21) Application number: 17926791.9
(22) Date of filing: 26.09.2017
(51) Int. Cl.: C07C 67/08, C07C 67/54, C07C 69/82, B01J 31/22, B01J 21/06

(54) **METHOD OF PREPARING ESTERS OF TEREPHTHALIC ACID**
VERFAHREN ZUR HERSTELLUNG VON ESTERN VON TEREPHTHALSÄURE
PROCÉDÉ DE PRÉPARATION D'ESTERS D'ACIDE TÉRÉPHTALIQUE

(43) Date of publication of application: 05.08.2020
(73) Proprietor: Public Joint Stock Company "Sibur Holding", Tobolsk, Tyumen region 626150 (RU)
(72) Inventor: NOSIKOV, Aleksei Aleksandrovich, Tomskaya obl. 634534 (RU); POPOVTSEV, Egor Evgenievich, Seversk Tomskaya obl. 636000 (RU); IGASHEVA, Varvara Petrovna, Tomskaya obl. 636213 (RU)
(74) Representative: Peters, Andreas
(86) International application number: PCT/RU2017/000708
(87) International publication number: WO 2019/066671

(56) References cited:
- WO-A1-2016/043616
- WO-A2-2011/079952
- CA-A1- 2 207 111
- US-A- 2 494 133
- US-A- 2 976 030
- US-A- 2 976 030
- US-A- 6 087 527
- US-A1- 2014 275 612
- US-B1- 9 688 838

## Description

The present invention relates to the production of ester plasticizers used in the manufacture of polyvinyl chloride (PVC) plastic materials. In particular, the invention relates to esters of terephthalic acids prepared by esterification of terephthalic acid with alcohols.

### Background

Methods of preparing terephthalic acid-based plasticizers by esterification of the acid with an alcohol are well known from the patent and other scientific literature. The steps of this process and their sequence are also well known, namely: the step of esterification, then distillation of an excess of alcohol, neutralization of acidic impurities, followed by drying and filtration.

Reactions of esterification are catalyzed reactions. The use of inorganic and organic metal-containing compounds, in particular titanium-containing compounds, as a catalyst is known in the prior art. High activity and selectivity of titanium-containing catalysts, their high solubility and, when they are used, the absence of acidic wastewater causing corrosion of equipment were the reasons for widespread use of these catalysts in industry.

For example, applications US20070161815 and WO2008094396 disclose a method of esterification of terephthalic acid (TPA) with an alcohol comprising from 6 to 10 carbon atoms, in the presence of titanium tetraisopropoxide (TTIP) as a catalyst in an amount of 0.2 wt.% under the atmospheric pressure, at a temperature of from 180 to 225°C for 8 to 9 hours. A drawback of this method is the long duration of the esterification process.

Patents CN102807491 and CN105130804 disclose a process of esterification of carboxylic acids with low-molecular weight alcohols comprising less than 7 carbon atoms to produce ester plasticizers or their compositions. The method according these patents also has drawbacks, in particular, a high temperature of from 210 to 230°C and higher, and a high pressure that is selected from 8 to 10 atm.

It is known that esterification in the presence of metal-containing catalysts is carried out at relatively high temperature and pressure, and often for a long period of time, which entails an increase in energy and raw material consumption. Active, low-cost, strong protonic acids are an alternative to metal-containing catalysts requiring high-energy consumption at the esterification step. Unlike metal-containing catalysts, protonic acids allow esterification to run faster at lower temperature and pressure with a high conversion of the initial acid. Sulfuric, phosphoric, nitric, p-toluenesulfonic, methanesulfonic, ethanesulfonic, propanesulfonic, butanesulfonic acids, various ion exchange resins and a combination thereof are used as acid catalysts for the esterification reaction.

However, protonic acids as catalysts are not devoid of significant drawbacks, such as low selectivity due to their ability to accelerate side reactions that, in turn, affect the color of the target product, and the necessity to neutralize the catalyst. In addition, the use of an acid catalyst results in corrosion of steel equipment used in the esterification process.

In the application CN104072365, the esterification reaction is performed in the presence of sulfuric acid as a catalyst at a ratio of sulfuric acid:alcohol of 1:3. This provides a high reaction rate (the duration of the reaction is from 4.5 to 5 hours) at a temperature of about 200°C. However, drawbacks associated with the formation of a large amount of by-products and, therefore, with difficulty in isolation of the target product remain unresolved. In addition, the application does not provide information about quality of the target product; in particular, there is no information on acid number and color of the finished product.

The acid number indicates the amount of unreacted carboxilic acid and characterizes the completeness of the reaction. A high content of acids leads to premature yellowing of articles, thus shortening their service life. The color of a plasticizer, in turn, affects the optical properties of the final product. A high color value of a plasticizer does not provide a transparent and snow-white final product, which significantly limits the field of application of the plasticizer.

Thus, a quality plasticizer must be characterized by as low value of color and acid number as possible.

All listed drawbacks associated with acid catalysts and metal-containing catalysts, both used alone, stimulated the search for new catalysts for the esterification reaction. Various alternatives of complex catalyst systems and methods of their introduction into a reactor are known from the prior art. Such systems, as a rule, make it possible to reduce a negative effect of a single catalyst and to achieve a synergistic effect in minimizing the formation of by-products, increasing the quality of the produced ester plasticizer, and also in intensifying the esterification process.

Thus, application WO201179952 provides a complex catalyst system consisting of a metal compound selected from titanium, tin, and zirconium; and an acid selected from sulfurous, phosphorous and/or hypophosphorous acid. The components of a two-component catalyst system are added to a reactor simultaneously. Thus, inventors of this application achieve an increase in the reaction rate and the possibility of carrying out the process under conditions with lower temperature (from 160 to 220°C) and pressure (from 4 to 7 atm.). However, according to the invention, the achievement of such a technical result requires sophisticated reactor equipment. Furthermore, the addition of acid catalysts and metal-containing catalysts at the beginning of the reaction leads to the formation of by-products (ethers) and, as a consequence, to a reduction in the yield of the final product.

As discussed above, the method of introducing a catalyst into a reactor has a significant effect on the esterification process. It should be noted that the methods of sequential (stepwise) introduction of catalysts have a number of advantages, in particular, the addition of optimal amounts of catalysts, an increase in the conversion rate, a reduction in the formation of by-products, and an increased process speed.

Patent CN102824929 suggests a multicomponent catalyst system for the esterification reaction and production of ester plasticizers, the catalyst system consisting of two catalysts each of which comprises a number of components. In one embodiment of the invention, a first catalyst is a mixture of an ionic liquid in an amount of 70 to 95 wt.% and a mental compound in an amount of 5 to 30 wt.%; and a second catalyst comprises 60-80% of a titanium-containing compound and 20-40% of another metal compound. The method of introducing the first and second catalysts is based on the sequential addition: the first catalyst is introduced into the reactor at the initial step of esterification, and the second catalyst is introduced when the temperature reaches 160-220°C. It is obvious that the complicity of such a catalyst system renders the system inapplicable in the large-scale production of plasticizers.

Document US 2976030 disclose a process for making dimethyl terephthalate which process comprises (1) intimately contacting terephthalic acid and methanol, in a weight ratio of methanol to acid of between about 2 and 5, at a temperature of about 240 C. at autogenous pressure for a time of about 20 minutes in the absence of a catalyst, (2) increasing rapidly the temperature of the reaction mixture from step 1 to a temperature of about 300 C. and maintaining said temperature, at autogenous pressure, for a time of about 20 minutes in the absence of a catalyst and (3) recovering dimethyl terephthalate from the reaction product mixture of step 2. There are no data on the composition of the products (diester and monoester), as well as data on the color and acidity of the ester. The inventors point out that the catalysts only accelerate the process but do not affect the process parameters.

A technical solution close to the present invention in terms of addition of catalysts and parameters of the process is the process for preparing plasticizer esters for polyvinylchloride (PVC), disclosed in patent US6355817. This process is characterized by the sequential addition of esterification catalysts, wherein a metal-containing catalyst is added in the first step, and a metal-containing catalyst other than the first catalyst is added in the second step. The total amount of the added catalyst system is 0.45 wt.%.

However, despite the similarity in the esterification process, there are significant differences, in particular the use of phthalic acid whose compounds are highly toxic, as well as an alcohol comprising more than 6 carbon atoms, whereas according to the present invention, it is necessary to use terephthalic acid and lower alcohols comprising 1 to 6 carbon atoms. In addition, the inventors of US6355817 are silent about the properties of the obtained product.

Thus, catalysts for esterification and methods of their introduction into a reactor, known in the prior art, do not provide a 100% conversion rate of TPA, a reduction in the formation of by-products, and a reduction in the duration of the process (about 4-5 hours), a decrease in temperature (180-200°C), and in pressure (4-7 atm.).

### Summary of the invention

The object of the invention is to provide a method of preparing esters of terephthalic acid with high conversion of the acid and a low content of by-products (dibutyl ether (DBE), mono-ether, p-toluenesulfonic acid butyl ester (pTSB)).

The technical result of the present invention is to increase the conversion of terephthalic acid up to 100% and to reduce the amount of by-products formed during the preparation of terephthalic acid esters, and, as a consequence, to increase the yield of the target product. An additional technical result is a low acid number (1.4 mg KOH/g), and a low value of the indicator characterizing the color of the resulting terephthalic acid ester.

The object is addressed and the technical result is achieved by using at the step of esterification running in a single reaction vessel, two sequentially added catalysts of different nature: a metal-containing catalyst added in the first step and an acid catalyst used in the second step of esterification.

The inventors unexpectedly have found that the use of a metal-containing catalyst in the first step and an acid catalyst in the second step of the esterification process makes it possible to obtain a product with a reduced amount of impurities, a low acid number and low color. In addition, a high conversion rate (100%) of terephthalic acid is achieved at a temperature of 180 to 200°C inclusive, a pressure of 4 to 7 atm. and a reaction time of 4 to 5.5 hours, which is optimal process conditions for a 100% conversion rate of TPA. The total amount of the catalyst system comprising a organometallic catalyst and an acid catalyst is not more than 0.7% by weight, which does not exceed the concentrations of catalysts (organometallic or acid catalysts used alone) commonly used in this technical field.

### Description of drawings

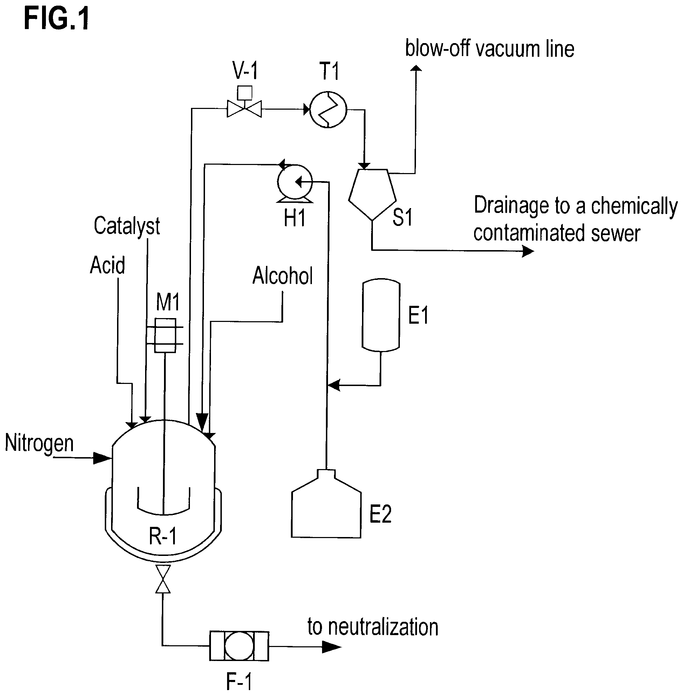

The technical solutions that disclose the essence of the invention are shown in detail in FIG.1 that is a schematic diagram of a plant for the production of a terephthalic acid ester.

The invention is explained in more detail with reference to FIG. 1 that shows a process flow diagram for the production of a terephthalic acid ester, wherein R-1 is an esterification reactor equipped with a heating element and mixing device M1, T-1 is a condenser, S-1 is a separator, E-1 is a container for feeding a second catalyst, E-2 is a container for dehydrated alcohol, and H-1 is a pump. The diagram in the figure is an exemplary embodiment of the invention and is not intended to limit thereof to particular details of the present disclosure.

A terephthalic acid ester is prepared by using the following sequential steps, wherein:
1) according to the inventive method, terephthalic acid, alcohol, a metal-containing catalyst, and optionally a sorbent are fed to esterification reactor R-1 equipped with a heating element and mixing device M-1;
2) further, the reaction mixture of the starting components in reactor R-1 is heated to the initial temperature of 160 to 185°C;
3) when the pressure in reactor R-1 reaches 4 to 7 atm (4-7 bar), valve V-1 is opened to release vapors of an azeotropic alcohol-water mixture;
4) the water vapors in the alcohol are delivered through a steam line to condenser T-1 where they condense and enter separator S-1;
5) in separator S-1, the mixture is separated into two phases: the lower aqueous phase and the upper organic phase;
6) the aqueous phase is regularly removed (drained) from separator S-1;
7) 2.5-4 hours after reaching a pressure of 6 atm. (6 bar) , a second catalyst, which is an acid catalyst, is added in the form of a solution in alcohol to reactor R-1 from container E-1 by using pump H-1;
8) to accelerate the process and increase the selectivity of the process of preparing the target product, the pressure in the system is maintained throughout the entire reaction at 4-7 atm. (4-7 bar) by smoothly controlling the heating of the reaction mixture to 185-220°C to the end of the reaction;
9) an excess of alcohol is maintained by pump H-1 that supplies absolute alcohol from container E-2;
10) 5-6 hours after reaching a pressure of 6 atm. (6 bar) , the heating of the reaction mixture is stopped;
11) 6-7 hours after reaching a pressure of 6 atm (6 bar) , the reaction mixture is discharged from reactor R-1;
12) the resulting ester is filtered, if necessary, from unreacted terephthalic acid residues and the acid catalyst through filter F-1; and
13) the filtered ester is delivered to further purification (neutralization, evaporation, and filtration).

Figs 2 and 3 are 1H NMR and 13C NMR spectra of the resulting titanium compound prepared in Example 7a.

Figs 4 and 5 are 1H NMR and 13C NMR spectra of the resulting titanium compound prepared in Example 8a.

The resulting product was identified by gas chromatography methods. The resulting product was colorless oily liquid - a terephthalic acid ester prepared by esterification of the acid with butanol that is characterized by a low content of by-products (DBE, mono-ether, p-TSB), a low acid number and low color.

### Detailed description of the invention

A detailed description of various aspects and embodiments of the present invention is given below.

In accordance with the present invention, the method of preparing a terephthalic acid ester comprises the following steps:
a) esterification of terephthalic acid with an alcohol comprising 1 to 6 carbon atoms, while continuously maintaining an excess of the alcohol in the system, wherein the esterification comprises two sequential steps: the first step is esterification in the presence of a metal-containing catalyst; and the second step is esterification in the presence of an acid catalyst, thus producing a crude ester, wherein both steps of esterification run in the same reaction vessel;
b) neutralization of the acid catalyst in the crude ester;
c) removal of low-boiling impurities from the crude ester; and
d) filtration and drying of the ester to obtain the terephthalic acid ester.

### Step a) of esterification of terephthalic acid with alcohol

For the preparation of esters according to the present invention, the starting compounds for the esterification reaction are terephthalic acid and an alcohol comprising from 1 to 6 carbon atoms. The esterification reaction is characterized by that it is carried out in a single reaction vessel, while maintaining an excess of the alcohol in the system, wherein the esterification reaction comprises two sequential steps: the first step is esterification in the presence of a metal-containing catalyst; and the second step is esterification in the presence of an acid catalyst.

The alcohol is a monoatomic alcohol comprising 1 to 6 carbon atoms, preferably methanol, ethanol, propanol, butanol, iso-butanol, more preferably butanol.

In a preferred embodiment, the used dicarboxylic aromatic acid is terephthalic acid of the formula:

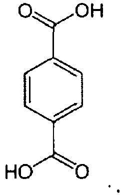

Esterification of terephthalic acid with an alcohol is carried out at a molar ratio of acid:alcohol of 1:1 to 1:3.

The esterification reaction of terephthalic acid with an alcohol is reversible, and it is preferable to carry out the esterification reaction while maintaining an excess of the alcohol in the system to shift the equilibrium of the reaction toward the formation of the target product. Thus, it is preferable to carry out esterification of terephthalic acid with an alcohol at a molar ratio of acid:alcohol of from 1:2 to 1:3, most preferably from 1:2.5 to 1:2.7.

The esterification reaction according to the present invention is characterized by that the process is carried out in two sequential steps:
step 1: esterification of terephthalic acid with an alcohol in the presence of a metal-containing catalyst at a temperature of 160 to 180°C inclusive;
step 2: addition of an acid catalyst after a certain period of time and continuation of the esterification reaction at a temperature of 180 to 220°C inclusive.

The metal-containing catalyst in the first step of the esterification reaction is an organo-inorganic compound containing a metal component, in particular titanium, tin, zirconium, etc. Titanium (IV) compounds, such as titanium tetraisopropoxide TTIP), titanium tetraisobutoxide, and compositions prepared by the reaction of titanium orthoester with an alcohol, acid and/or base are preferred. The titanium (IV) compound may be a compound of the general formula:

Tiₙ(OR)ₓ(OR')ₓO_{y}

wherein
n is an integer from 1 to 4;
y is an integer from 0 to 6;
x may be the same or different and is an integer from 2 to 8;
R is a linear or branched C₁-C₁₈alkyl, C₃-C₁₈cycloalkyl, R' is aryl optionally comprising an electron-donor substituent;
or a mixture thereof,
with proviso that
if n is 1, then x is 2 and y is 0; and
if n>1, then the compounds comprise at least two alkoxy groups and two aryloxy groups.

The titanium (IV) compound also may be a compound of formula I or II: 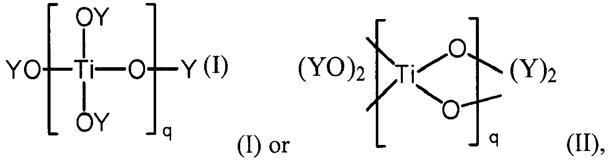 wherein
q is an integer from 1 to 4;
Y independently is R or R'
R is a linear or branched C₁-C₁₈alkyl, C₃-C₁₈cycloalkyl, R' is aryl optionally comprising an electron-donor substituent;
or a mixture thereof, with proviso that the compounds comprise at least two alkoxy groups and two aryloxy groups.

The C₁-C₁₈alkyl preferably may be methyl, ethyl, n-propyl, isopropyl, n-butyl, sec-butyl, isobutyl, tert-butyl, n-pentyl, sec-pentyl, isopentyl, tert-pentyl, neo-pentyl, hexyl, isohexyl, neo-hexyl, sec-hexyl, or tert-hexyl;
the C₃-C₁₈cycloalkyl preferably is C₅-C₇cycloalkyl, more preferably cyclopentyl, cyclohexyl, methycyclopentyl, methylcyclohexyl, dimethylcyclopentyl, ethylcyclopentyl, or cycloheptyl.

The electron-donor substituent of the aryl group preferably is selected from Ci-C₆alkyl, aryl, C₁-C₆alkoxy, C₁-C₆alkylamino, or C₁-C₆alkylthio.

The alkyl part in the electron-donor substituent preferably is methyl, ethyl, n-propyl, isopropyl, n-butyl, sec-butyl, isobutyl, tert-butyl, n-pentyl, sec-pentyl, isopentyl, tert-pentyl, 2,2-dimethylpentyl, hexyl, isohexyl, 2,2-dimethylbutyl, sec-hexyl, or tert-hexyl.

Aryl preferably is phenyl.

The number of alkoxy and aryloxy groups in the titanium (IV) compound may be the same or different and may be from 2 to 8 groups.

In a preferred variant, the number of alkoxy and aryloxy groups in the titanium (IV) compound is the same and is from 2 to 5 groups.

The number of alkoxy and aryloxy groups in the titanium (IV) compound may be different and independently may be 2, 3, 4, 5, 6, 7, or 8 groups depending on the number of titanium and oxygen atoms in the molecule.

The maximum number of aryloxy and alkoxy groups in the titanium (IV) compound with one titanium atom may be 4, with two titanium atoms - 6, with three titanium atoms - 8, and with four titanium atoms - 10.

The titanium (IV) compound may exist in different structural forms, in particular, in the form of mono-, di-, tri- or tetramers.

Preferred titanium (IV) compounds used in the present invention are compounds with the following structures:

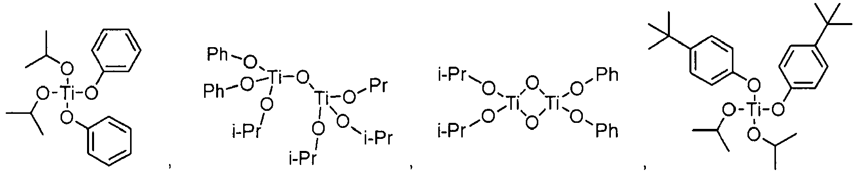

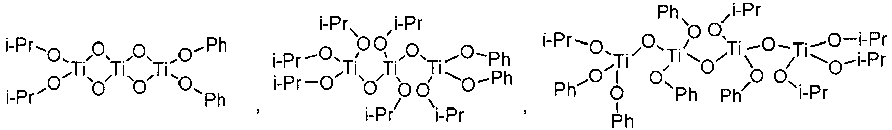

Said titanium (IV) compounds according to the present invention are prepared from chemically pure raw materials which are phenol or its derivatives and tetraalkyl titanates in an organic solvent, preferably from dehydrated toluene in an inert gas, preferably in nitrogen, by adding to the solution a hydroxyaromatic compound, preferably phenol or a derivative thereof, in an organic solvent for tetraalkyl titanate with stirring at an elevated temperature, preferably at 60 to 90°C.

The reaction time is several hours, preferably from 10 to 12 hours, followed, if necessary, by removal of the solvent under a reduced pressure.

The amount of the used metal-containing catalyst is from 0.1 to 1 wt.% inclusive, preferably from 0.5 to 0.6 wt.%.

The addition of an acid catalyst in the second step of the process of esterification of terephthalic acid with an alcohol comprising from 1 to 6 carbon atoms, in the presence of a metal-containing catalyst is essential to achieve the claimed technical result.

The acid catalyst may be any esterification catalyst known in the prior art. Examples of such catalysts include, but are not limited to, sulfuric acid, p-toluenesulfonic acid (p-TSA), methanesulfonic acid, etc. The acid catalyst in the second step of esterification preferably is p-toluenesulfonic acid.

The amount of the used acid catalyst is from 0.1 to 0.5 wt.%, preferably from 0.2 to 0.3 wt.%.

In the esterification process, the total amount of the catalyst system comprising an organometallic catalyst and an acid catalyst is from 0.2 to 1.5 wt.%, preferably from 0.7 to 0.9 wt.%, most preferably 0.7 wt.%.

**The acid catalyst is added at the second esterification step 2.5 to 5.5 hours,** preferably 3.0 to 4.0 hours, after heating the reaction mixture comprising terephthalic acid and alcohol.

The addition of the acid catalyst before 2.5 hours from the beginning of the esterification step leads to an increase in by-products and a reduction in the yield of the target product. The addition of the acid catalyst after 5.5 hours from the beginning of the esterification step does not provide a 100% conversion rate of terephthalic acid.

The temperature at the esterification step depends on the nature and concentration of the used catalyst, in particular when in the first step, an acid catalyst is used, such as p-TSA, a sufficient temperature of the process is in the range of 160 to 185°C inclusive. In case of a metal-containing catalyst, the temperature should be increased from 185 to 220°C inclusive. To shorten the time of the process, it is preferable to start the heating during the loading of the starting components: terephthalic acid, an alcohol, and a metal-containing catalyst.

According to the present invention, when using two catalysts of different nature, for example TTIP and p-TSA, the esterification reaction is carried out under heating the reaction mixture from 180°C (inclusive) in the first step up to 200°C (inclusive) in the second step of esterification.

The pressure at the esterification step is from 4 to 7 atm (4-7 bar) , preferably from 5 to 6 atm., (5-6 bar), most preferably 6 atm. (6 bar).

It is preferable when the esterification reaction is carried out in an inert gas atmosphere to avoid thermal-oxidative processes. The inert gas may be argon, nitrogen, helium, carbon dioxide, etc.

According to the present invention, the esterification process is performed in a single reaction vessel, which is a reactor equipped with a heating element, a mixing device in the form of a mixer or a circulation pump, and a vapor delivery tube used for the removal of the reaction water from the reactor in the form of an azeotropic mixture with an alcohol.

The duration of the esterification step is from 3.5 to 9 hours, inclusive, preferably from 5 to 7 hours, most preferably 6 hours.

The starting components in the first step (terephthalic acid, an alcohol, a metal-containing catalyst, and an optional adsorbent) are added to a reactor in any order, both separately and in combination. An acid catalyst in the second step is added 2.5 to 5 hours, preferably 3.0 to 4.0 hours, after the start of the heating of the starting components.

The components preferably are added in the following sequence: 1) terephthalic acid; 2) an alcohol; 3) a metal-containing catalyst; and 4) an acid catalyst 3.5 to 5 hours after the start of heating. Terephthalic acid is added in powdered form. The metal-containing catalyst is added to the reactor in pure form, in the form of a suspension or a solution, in a required amount, in whole or portionwise, in two or more portions. The acid catalyst is added to the reactor in pure form, in the form of a solution in an alcohol, in whole or portionwise, in two or more portions.

In order to reduce the color of the target product, in the esterification step, adsorbents are used that are capable of adsorbing coloring impurities resulting from chemical transformations of terephthalic acid. The adsorbents include, in particular, activated carbon, zeolite, perlite, etc. The amount of the adsorbent is from 0.01 to 2 wt.% inclusive, preferably from 0.1 to 1 wt.%. The adsorbent is added to the esterification reactor separately or in combination with the other starting components.

In accordance with the present invention, during the esterification step, it is necessary to remove from the reactor an azeotropic mixture in the form of water and alcohol because of a high solubility of water in alcohol (20% by weight of water in butanol) and a possible hydrolysis of the metal-containing catalyst in the presence of water and a reduction of its catalytic activity. However, the need to maintain an excess of alcohol during the esterification reaction should be taken into account.

Thus, the maximum effective use of the catalyst resource, its long-term efficiency, and the maximum conversion of terephthalic acid are achieved under the following conditions: 1) removal of water from the system by withdrawal of watered alcohol during the esterification reaction; and 2) maintaining a required excess of alcohol in the system.

The required excess of alcohol can be maintained in the system by adding to the reaction vessel an additional amount of alcohol delivered from outside, for example, from a separate container. In order to avoid a negative action of water contained in alcohol on the metal-containing catalyst, it is preferable to use an alcohol with a low content of water, i.e. absolute (anhydrous) alcohol.

Another alternative method of maintaining the required excess of alcohol in the system is to introduce into the ester production process an additional step of separation of the alcohol:water azeotropic mixture and subsequent recycling of the alcohol into the esterification reaction. In this case, the alcohol :water azeotropic mixture is separated in an apparatus for separation of liquids, in particular in a Florentine vessel.

The reaction mass obtained in the above-discussed esterification step comprises: a crude ester, a hydrolyzed metal-containing catalyst, an acid catalyst, residual amounts of the alcohol and water, and adsorbent, if used.

### Step b) of neutralization of an acid catalyst

The step of neutralization is necessary to remove the catalyst from the reaction mixture obtained at the esterification step. This step is carried out by using a neutralizing agent, which may be an alkaline aqueous solution.

The alkaline aqueous solution, as used herein, means solutions with pH of more than 7, in particular solutions of the following compounds: sodium hydroxide, sodium, potassium or magnesium carbonate, sodium silicate, potassium hydroxide, etc.

The amount of the neutralizing agent usually ranges from 0.5 to 6 moles per mole of acid compound, preferably from 3 to 4 moles per mole of acid compound.

The neutralization step preferably is carried out at a temperature of from 50 to 90°C inclusive and under the atmospheric pressure.

At the end of the neutralization step, the reaction mass comprises a crude ester, a hydrolyzed metal-containing catalyst, salts of acids, residual amounts of the alcohol and water, and adsorbent, if used.

### Step c) of removal of low-boiling impurities

The purification of the reaction mass comprising the crude ester also includes the removal of low-boiling impurities - alcohol and water. The low-boiling impurities are removed by any method known in the prior art, in particular by rectification, evaporation, and the like.

According to the present invention, the low-boiling impurities (water and alcohol) are removed by rectification in a column apparatus, which is a tray-type rectifying column. The alcohol purified from water is recycled into the esterification reaction.

According to the present invention, the apparatus works in an bubble mode and is intended for the removal of an excess of alcohol and low-boiling impurities from the neutralized ester by azeotropic distillation with water vapor.

### Step d) of filtration and drying

The product resulting from steps (a)-(c), which is a crude ester, is filtered to separate the adsorbent, if used, and residues of the catalyst hydrolyzed at the step of catalyst neutralization. The filtration step is carried out in any apparatuses known in the prior art, such as filters equipped with porous filters capable of separating the solid phase from the target product. The ester is filtered at a temperature of from 50 to 90°C inclusive.

The produced ester is dried to remove water and/or vapors thereof. The drying step may be carried out by physical techniques usually used for separation and purification of organic compounds (extraction, desalting, fractional and azeotropic distillation, evaporation, etc.) and by using drying agents that remove moisture by adsorption, through the formation of hydrates or chemical reactions with water.

The drying is preferably performed at a temperature of from 105 to 115°C inclusive and under a pressure of from 0.05 to 0.1 atm.

The product prepared according to the present invention is an ester of terephthalic acid and an alcohol comprising from 1 to 6 carbon atoms. The resulting product is characterized by a low acid number and low color and may be used as a plasticizer for polyvinyl chloride (PVC). The acid number, as used herein, is the amount (in milligrams) of potassium hydroxide (KOH) required for neutralization of all acidic components comprised in 1 g of the obtained ester. The color, as used herein, is a quality index of the resulting ester, characterizing the intensity of the color caused by the presence of coloring compounds.

### Embodiments of the invention

In the examples below, the properties of the obtained esters were studied by the following methods:
1) identification of DBTP ester is based on the gas chromatography analysis;
2) the acid number determination method is based on the reaction of neutralization of residual acidity in a plasticizer with an alkaline solution according to National Standard - GOST 8728-88 (6-7 pages, 3.6 item).
3) The color of the ester was determined according to ASTMD 3-05.

### Example 1. Esterification by sequential introduction of titanium tetraisopropoxide (TTIP) and then para-toluenesulfonic acid (p-TSA)

A 15 liter steel reactor equipped with a heating element and a stirring device was charged with 3000 grams of terephthalic acid, 3500 grams of butanol, 32.5 grams of titanium (IV) tetraisopropoxide.

In addition, 2300 grams of butanol were added to a separate container for deliver thereof to the reactor during the esterification process.

The reaction was carried out in the flow of an inert gas, which was nitrogen, at a flow rate of 25 L/h.

The reaction mass was heated to 180°C, and the pressure in the system was 6 atm. Three hours after reaching a pressure of 6 atm., a solution of p-TSA (13 g) in butanol was added to the reactor by using a pump.

The alcohol evaporated during the reaction was condensed and collected in the additional container. The required excess of alcohol was maintained by its delivery from the separate container.

The pressure in the reactor during the esterification process was 6 atm., and the temperature was continuously raised to 200°C. The total reaction time was 6 hours.

After six hours, the reaction mass was filtered and cooled.

6220 grams of the reaction mass were discharged from the esterification reactor, the mass containing 0.3 wt.% dibutyl ether, 0.06 wt.% butyl ester of para-toluenesulfonic acid, 0 wt.% butylterephthalate, 19.08 wt.% butanol, and 79.97 wt.% dibutyl terephthalate. Conversion of TPA was 100%.

### Example 2. Esterification with a para-toluenesulfonic acid (p-TSA) catalyst

The process was carried out as disclosed in Example 1, except that para-toluenesulfonic acid was used alone as a catalyst in an amount of 32.5 grams (0.5 wt.%). After the esterification reaction, the reaction mass was also filtered from TPA residues.

After six hours, 6200 grams of the reaction mass were discharged from the esterification reactor, the mass containing 2.1 wt.% dibutyl ether, 0.20 wt.% butyl ester of para-toluenesulfonic acid, 2.1 wt.% butylterephthalate, 18 wt.% butanol, and 76.8 wt.% dibutylterephthalate. Conversion of TPA was 100%.

### Example 3. Esterification with a titanium tetraisopropoxide (TTIP) catalyst

A difference from Example 1 is the use of titanium (IV) isopropoxide alone as a catalyst in an amount of 32.5 grams (0.5 wt.%). After the esterification reaction, the reaction mass was filtered from TPA residues.

After six hours, 6200 grams of the reaction mass were discharged from the esterification reactor, the mass containing 0.069 wt.% dibutyl ether, 0 wt.% butyl ester of para-toluenesulfonic acid, 0 wt.% butylterephthalate, 38.94 wt.% butanol, and 60.99 wt.% dibutylterephthalate. Conversion of TPA was 82.5%.

### Example 4. Esterification by sequential introduction of para-toluenesolfonic acid (p-TSA) and then titanium tetraisopropoxide (TTIP)

A difference from Example 1 is in that p-TSA was used as the first catalyst in an amount of 16.5 grams (0.25 wt.%) and then, after 3 hours, titanium (IV) isopropoxide was added in an amount of 16,25 grams (0.25 wt.%). After the esterification reaction, the reaction mass was also filtered from TPA residues.

After six hours, 5000 grams of the reaction mass were discharged from the esterification reactor, the mass containing 0.22 wt.% dibutyl ether, 0.25 wt.% butyl ester of para-toluenesulfonic acid, 0.51 wt.% butylterephthalate, 17.75 wt.% butanol, and 81.27 wt.% dibutylterephthalate. Conversion of TPA was 80.6%.

### Example 5. Esterification by sequential introduction of titanium tetraisopropoxide (TTIP) at the first step of the reaction and then TTIP at the second step of the reaction (according to prototype US6355817)

A difference from Example 1 is in that TTIP was used as the first step catalyst in an amount of 16.25 grams (0.25 wt.%) and then, after 3 hours, the second portion of TTIP was added in an amount of 16.25 grams (0.25 wt.%). After the esterification reaction, the reaction mass was filtered from TPA residues.

After six hours, 3300 grams of the reaction mass were discharged from the esterification reactor, the mass containing 0.014 wt.% dibutyl ether, 0 wt.% butyl ester of para-toluenesulfonic acid, 0 wt.% butylterephthalate, 17.20 wt.% butanol, and 82.78 wt.% dibutylterephthalate. Conversion of TPA was 52.6%.

### Example 6. Esterification by sequential introduction of titanium tetraisopropoxide (TTIP) and then, after 6 hours, para-toluenesulfonic acid (p-TSA)

A difference from Example 1 is in that TTIP was used as the first step catalyst in an amount of 32.5 grams (0.5 wt.%) and then, after 6 hours, p-TSA was added in an amount of 16.25 (0.25 wt.%). After the esterification reaction, the reaction mass was also filtered from TPA residues.

At the end of the reaction, 5127 grams of the reaction mass were discharged from the esterification reactor, the mass containing 0.30 wt.% dibutyl ether, 0.21 wt.% butyl ester of para-toluenesulfonic acid, 0 wt.% butylterephthalate, 35.63 wt.% butanol, and 63.86 wt.% dibutylterephthalate. Conversion of TPA was 82.7%.

### Example 7a. Preparation of a titanium (IV) compound - an metal-containing catalyst for esterification

The initial reagents, namely phenol and titanium tetraisopropoxide, were purified by vacuum distillation.

Phenol (184.6 mmol) and 260 ml of dehydrate toluene were loaded into a 500 ml two-necked flask equipped with a stirrer and filled with dry nitrogen. TTIP (92.2 mmol) was added to the obtained solution in a single portion. The obtained solution was stirred at 80°C for 12 hours under an inert gas atmosphere.

Toluene was removed in a rotary evaporator at 80°C and under a pressure of 100 to 2 mm Hg to obtain tar-like compound of red-orange color.

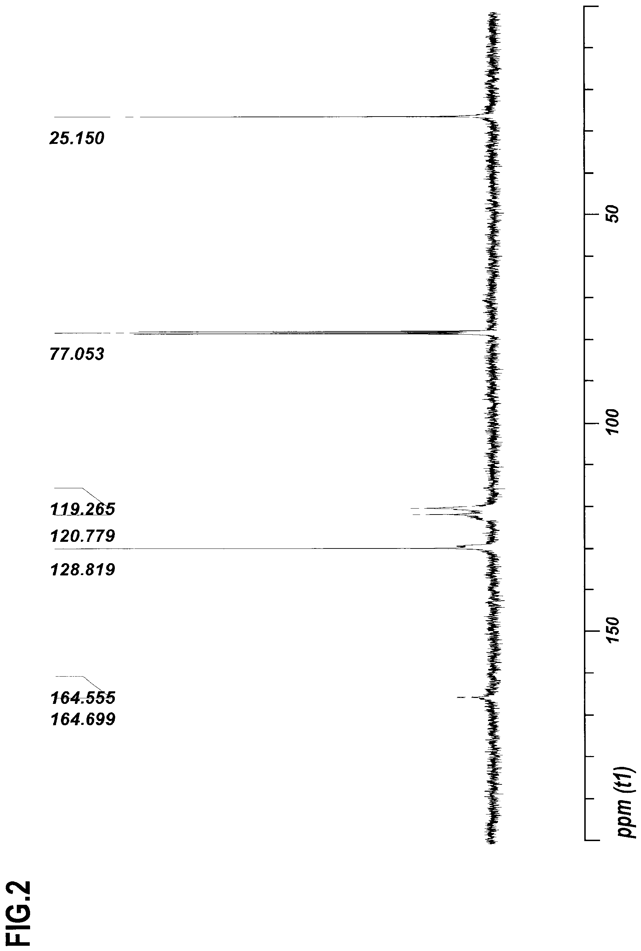

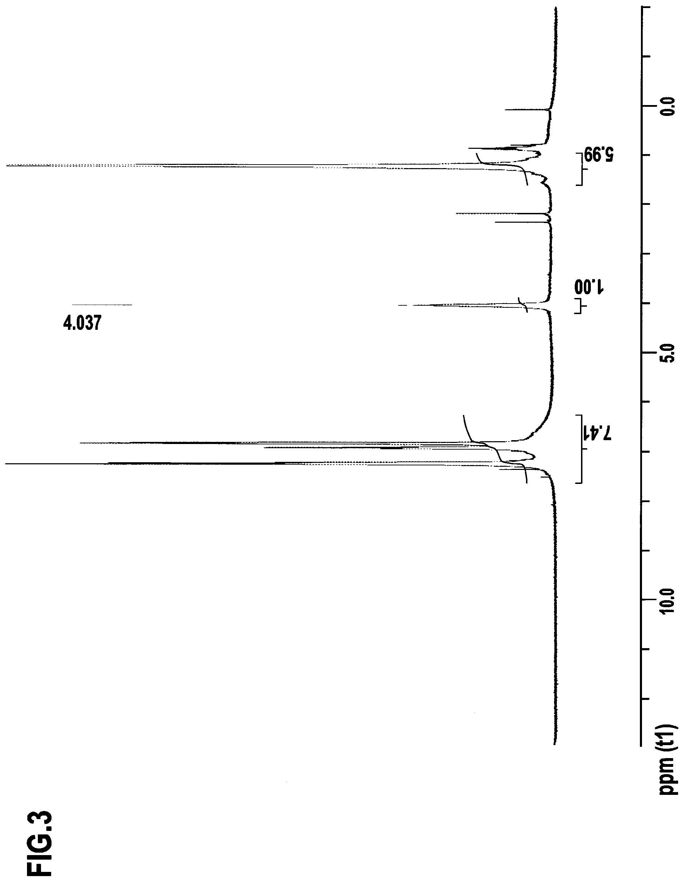

1H NMR and 13C NMR spectra of the obtained compound are shown in Figs 2 and 3.

### Example 7b. Esterification by sequential introduction of an metal-containing catalyst (the titanium (IV) compound prepared according to Example 7a) and then, after 3 hours, para-toluenesofonic acid (p-TSA)

A difference from Example 1 is in that an metal-containing catalyst (the titanium (IV) compound prepared according to Example 7a) was used as the first step catalyst in an amount of 32.5 grams (0.5 wt.%) and then, after 3 hours, p-TSA was added in an amount of 13 grams (0.2 wt.%).

After the esterification reaction, the reaction mass was also filtered.

6240 grams of the reaction mass were discharged from the esterification reactor, the mass containing 0.12 wt.% dibutyl ether, 0 wt.% butyl ester of para-toluenesulfonic acid, 0 wt.% butylterephthalate, 19.67 wt.% butanol, and 80.21 wt.% dibutylterephthalate. Conversion of TPA was 100%.

### Example 8a. Preparation of a titanium (IV) compound - an metal-containing catalyst for esterification

n-Tert-butylphenol (139.93 mmol) and 200 ml of dehydrate toluene were loaded into a 500 ml two-necked flask equipped with a stirrer and filled with dry nitrogen. TTIP (69.98 mmol) was added to the obtained solution in a single portion. The obtained solution was stirred at 80°C for 12 hours under an inert gas atmosphere.

Toluene was removed in a rotary evaporator at 80°C and under a pressure of 100 to 2 mm Hg to obtain tar-like compound of red-orange color.

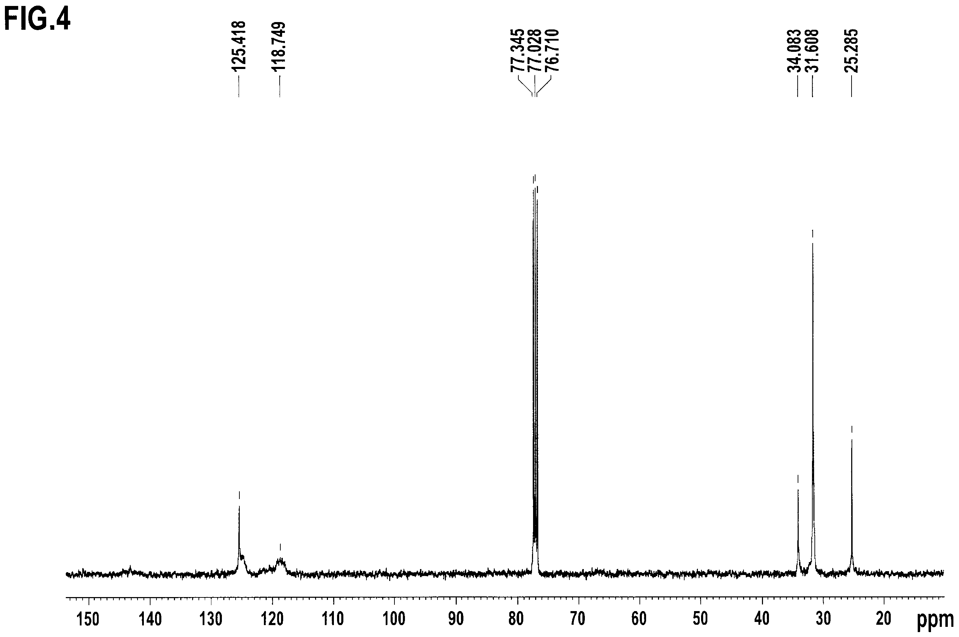

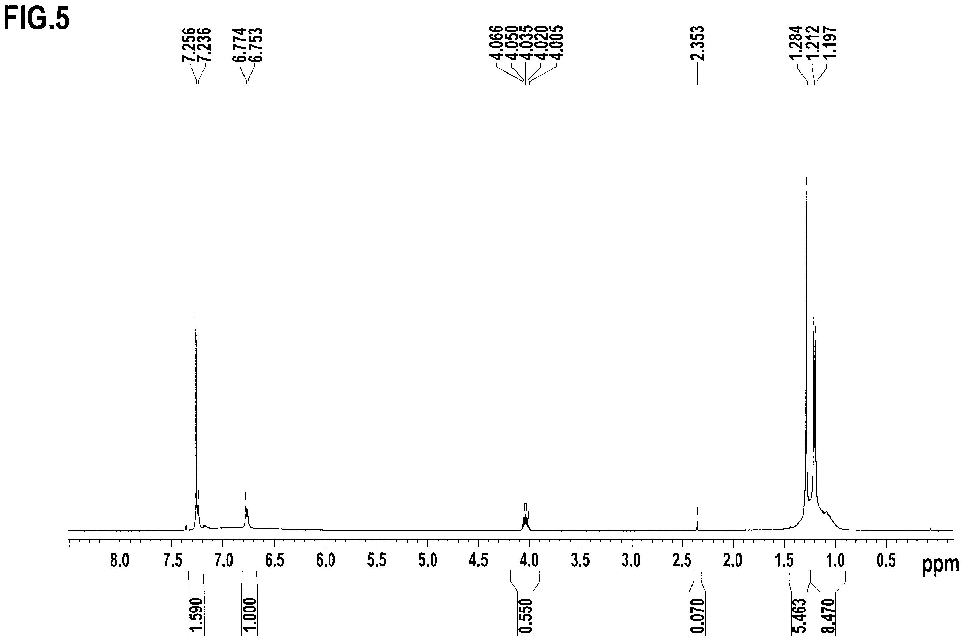

1H NMR and 13C NMR spectra of the obtained compound are shown in Figs 4 and 5.

### Example 8b Esterification by sequential introduction of an metal-containing catalyst (the titanium (IV) compound prepared according to Example 8a) and then, after 3 hours, para-toluenesofonic acid (p-TSA)

A difference from Example 1 is in that an metal-containing catalyst (the titanium (IV) compound prepared according to Example 8a) was used as the first step catalyst in an amount of 32.5 grams (0.5 wt.%) and then, after 3 hours, p-TSA was added in an amount of 13 grams (0.2 wt.%).

After the esterification reaction, the reaction mass was also filtered.

6150 grams of the reaction mass were discharged from the esterification reactor, the mass containing 0.15 wt.% dibutyl ether, 0 wt.% butyl ester of para-toluenesulfonic acid, 0 wt.% butylterephthalate, 18.51 wt.% butanol, and 81.34 wt.% dibutylterephthalate. Conversion of TPA was 100%.

Results of the esters prepared according to Examples 1-7 are shown in Table.

**Table. Data on the conversion of TPA, weight content of the components (wt.%), and acid number.**

| Example, No | Product yield, % | Conversion of TPA, % | DBTP selectivity, % | Appearance of the ester/color, Hazen units | Esterification | | | | | |
|---|---|---|---|---|---|---|---|---|---|---|
| | | | | | Weight content of the components, wt.% | | | | | Acid number, mg KOH/g |
| | | | | | n-butanol | DBE | p-TSBE | Mono BTP | DBTP | |
| Example 1 | 96.8 | 100.0 | 96.8 | Colorless oily liquid/ 15 | 19.08 | 0.9 | 0.06 | - | 79.97 | 1.4 |
| Example 2 | 91.5 | 100.0 | 91.5 | Oily liquid with a greenish tinge/ 120 | 18.0 | 2.1 | 0.20 | 2.1 | 76.80 | 12.6 |
| Example 3 | 77.1 | 82.5 | 93.4 | Colorless oily liquid/ 30 | 38.94 | 0.069 | - | - | 60.99 | 8.7 |
| Example 4 | 79,5 | 80.6 | 98,6 | Oily liquid with a greenish tinge/ 80 | 17.75 | 0.22 | 0.25 | 0.51 | 81.27 | 6.2 |
| Example 5 | 50,1 | 52.6 | 95.3 | Colorless oily liquid/ 30 | 17.20 | 0.014 | - | - | 82.78 | 8.5 |
| Example 6 | 78,1 | 82.7 | 94,5 | Colorless oily liquid/ 30 | 35.63 | 0.30 | 0.21 | ND | 63.86 | 6.4 |
| Example 7b | 99.7 | 100.0 | 99.7 | Colorless oily liquid/ 30 | 19.67 | 0.12 | - | ND | 80.21 | 1,5 |
| Example 8b | 99.6 | 100.0 | 99.6 | Colorless oily liquid/ 30 | 18.51 | 0.15 | - | ND | 81.34 | 1,3 |

| | | | | | | | | | | |
|---|---|---|---|---|---|---|---|---|---|---|
| Example 1 Preparation of an ester in the presence of an metal-containing (TTIP) catalyst and an acid (p-TSA) catalyst Example 2 Preparation of an ester in the presence of an acid (p-TSA) catalyst alone Example 3 Preparation of an ester in the presence of an metal-containing (TTIP) catalyst alone Example 4 Preparation of an ester by using first an acid (p-TSA) catalyst and then an metal-containing (TTIP) catalyst Example 5 Preparation of an ester by using at both steps of esterification an metal-containing (TTIP) catalyst Example 6 Preparation of an ester by using an metal-containing (TTIP) catalyst and an acid (p-TSA) catalyst added six hours after the beginning of esterification Example 7b Preparation of an ester by using first an metal-containing catalyst prepared according to Example 7a and then an acid (p-TSA) catalyst added six hours after the beginning of esterification Example 8b Preparation of an ester by using first an metal-containing catalyst prepared according to Example 8a and then an acid (p-TSA) catalyst added six hours after the beginning of esterification | | | | | | | | | | |

The data of Example 1 show that the combined use of a metal-containing catalyst and an acid catalyst makes it possible to obtain a product with required parameters, in particular with a low impurity content and a 100% conversion rate of terephthalic acid. It was also unexpectedly found that the combined use of the catalysts leads to a significant reduction in the acid number to 1.4 mg KOH/g, and to the production of a low-color ester. The total amount of the catalyst system comprising an organometallic catalyst and an acid catalyst is 0.7% by weight and does not exceed the concentrations of the "individual" catalysts (an organometallic catalyst alone or an acid catalyst alone) commonly used in this technical field.

The data of Example 2 show that the use of an acid catalyst alone leads to an increase in conversion of the acid, but the amount of impurities in the product increases. The acid number, in this case, is 12.6 mg KOH/g, and the resulting product has a high value of color.

The data of Example 3 show that the use of a metal-containing catalyst alone leads to a reduction in the amount of impurities in the product, but a 100% conversion rate of the acid is not achieved. The acid number is 8.7 mg KOH/g. It should be noted that the acid number was determined after filtration of the obtained ester and removal of unreacted terephthalic acid from its solution. Since terephthalic acid is slightly soluble (0.1% in methanol at 25°C), its contribution to the OH group content in the solution and, as a consequence, its effect on the acid number are negligible.

The data of Example 4 show that the addition of an acid catalyst in the first step of the esterification process leads to an increase in the impurity content; moreover, the conversion does not reach 100%, the product color is unsatisfactory, and the acid number is 6.2 mg KOH/g, which confirms that the order of the steps of the catalyst introduction, as indicated in Example 1, is important.

The data of Example 5 show that the portionhwise addition of an organometallic catalyst does not provide the maximum conversion of the acid. The acid number after filtration of the product and removal of unreacted acid from the solution is 8.5 g KOH/g. It should be noted that the sample was analyzed for the acid number after filtration and removal unreacted acid from the solution.

The data of Example 6 show that the addition of an acid catalyst at the end of the esterification reaction (6 hours after the start of the reaction) leads to a reduction in the impurity content in the product, but 100% conversion of the acid is not achieved. The acid number is 6.4 mg KOH/g.

Thus, it has been shown that the best technical result is achieved only when at the first step of the esterification reaction, a metal-containing catalyst is used, and an acid catalyst is used at the second step. In addition, it is preferable to add an acid catalyst 2.5 to 5.5 hours, preferably 3.0 to 4.0 hours, after the beginning of the esterification step, i.e., after the start of the heating of terephthalic acid and alcohol.

## Claims

1. A method of preparing an ester of terephthalic acid, comprising the steps of:
a) esterification of terephthalic acid with an alcohol comprising 1 to 6 carbon atoms, while continuously maintaining an excess of the alcohol in the system, wherein the esterification step comprises two sequential steps: the first step is esterification by using a metal-containing catalyst; and the second step is esterification by using an acid catalyst, to produce a crude ester;
b) neutralization of the acid catalyst in the crude ester;
c) removal of low-boiling impurities from the crude ester;
d) filtration and drying of the ester to obtain the ester of terephthalic acid.

2. The method according to claim 1, wherein the esterification is carried out in a single reaction vessel, which is any reactor known in the prior art, equipped with a heating element.

3. The method according to claim 1, wherein the alcohol comprising 1 to 6 carbon atoms is a monoatomic alcohol comprising preferably 2 to 5 carbon atoms, most preferably 3 to 4 carbon atoms, most preferably butanol.

4. The method according to claim 1, wherein a ratio of terephthalic acid:alcohol is from 1:1 to 1:3, preferably from 1:2 to 1:3, most preferably 1:2.5 to 1:2.7.

5. The method according to claim 1, wherein the metal-containing catalyst added at the first step is a compound of titanium, tin, or zirconium, preferably a titanium (IV) compound, most preferably titanium tetraisopropoxide, titanium tetraisobutoxide, or a composition prepared by a reaction of titanium orthoester with an alcohol, acid and/or base.

6. The method according to claim 1, wherein an amount of the metal-containing catalyst is from 0.1 to 1 wt.%, preferably from 0.5 to 0.6 wt.%.

7. The method according to claim 1, wherein the acid catalyst added at the second step is a compound selected from the group of sulfur acid, methanesulfonic acid, para-toluenesulfonic acid, preferably para-toluenesulfonic acid.

8. The method according to claim 1, wherein an amount of the acid catalyst is from 0.1 to 0.5 wt.%, preferably from 0.2 to 0.3 wt.%.

9. The method according to claim 1, wherein the acid catalyst is added at the second step 2.5 to 5.5 hours, preferably 3.0 to 4.0 hours, after the beginning of the esterification step, i.e., after the start of the heating of terephthalic acid and the alcohol.

10. The method according to claim 1, wherein a temperature of esterification is 160 to 185°C inclusive at the first step, and 185 to 220°C inclusive at the second step.

11. The method according to claim 1, wherein the duration of the esterification step is 3.5 to 9 hours, preferably from 4 to 7 hours, most preferably 5 to 6 hours.

12. The method according to claim 1, wherein a pressure at the esterification step is from 4 to 7 atm. (4-7 bar), preferably from 5 to 6 atm. (5-6 bar), most preferably 6 atm. (6 bar).

13. The method according to claim 1, wherein an adsorbent is used.

14. The method according to claim 13, wherein the adsorbent is activated carbon, zeolite, or perlite.

15. The method according to claim 13, wherein an amount of the used adsorbent is from 0.01 to 2 wt.%, preferably from 0.1 to 1 wt.%.

16. The method according to claim 1, wherein the step of esterification comprises removal of an alcohol-water azeotropic mixture.

17. The method according to claim 1, wherein an excess of alcohol is maintained by separation of an alcohol-water azeotropic mixture, followed by recycling the alcohol into the esterification step.

18. The method according to claim 1, wherein an excess of alcohol is maintained by adding anhydrous alcohol from a separate container.

19. The method according to claim 1, wherein at the neutralization step, a neutralizing agent is used, which is a solution of sodium hydroxide; sodium, potassium, or magnesium carbonate; sodium silicate; potassium hydroxide; or the like.

20. The method according to claim 19, wherein an amount of the neutralizing agent is from 0.5 to 6 moles per mole of unreacted acid, preferably from 3 to 4 moles per mole of unreacted acid.

21. The method according to claim 1, wherein the step of neutralization is carried out at a temperature of from 50 to 90°C inclusive.

22. The method according to claim 1, wherein low-boiling impurities are removed in a column apparatus.

23. The method according to claim 22, wherein the apparatus works in an bubble mode.

24. The method according to claim 1, wherein the step of the ester filtration is carried out at a temperature of from 50 to 90°C.

25. The method according to claim 1, wherein the step of drying is carried out at a temperature of from 105 to 115°C inclusive.

26. The method according to claim 1, wherein the step of drying is carried out under a pressure of from 0.05 to 0.1 atm. (0.05 to 0.1 bar).

27. The method according to claim 1, comprising the steps of:
a) esterification of terephthalic acid with an alcohol comprising 1 to 6 carbon atoms, while continuously maintaining an excess of the alcohol in the system, wherein the esterification comprises two sequential steps:
the first step is esterification by using as a metal-containing catalyst a titanium (IV) compound of the general formula:
Tiₙ(OR)ₓ(OR')ₓO_{y}
wherein
n is an integer from 1 to 4;
y is an integer from 0 to 6;
x may be the same or different and is an integer from 2 to 8;
R is a linear or branched C₁-C₁₈alkyl, C₃-C₁₈cycloalkyl, R' is aryl optionally comprising an electron-donor substituent;
or a mixture thereof,
with proviso that
if n is 1, then x is 2 and y is 0; and,
if n>1, then the compounds comprise at least two alkoxy groups and two aryloxy groups;
and the second step is esterification by using an acid catalyst, to produce a crude ester;
b) neutralization of the acid catalyst in the crude ester;
c) removal of low-boiling impurities from the crude ester;
d) filtration and drying of the ester to obtain the ester of terephthalic acid.

28. The method according to claim 27, wherein R' is phenyl optionally comprising an electron-donor substituent, preferably the electron-donor substituent is an C₁-C₆alkyl, aryl, C₁-C₆alkoxy, C₁-C₆dialkylamino, or C₁-C₆alkylthio group, most preferably the alkyl part in the electron-donor substituent is preferably methyl, ethyl, n-propyl, isopropyl, n-butyl, sec-butyl, isobutyl, tert-butyl, n-pentyl, sec-pentyl, isopentyl, tert-pentyl, 2,2-dimethylpentyl, hexyl, isohexyl, 2,2-dimethylhexyl, sec-hexyl, or tert-hexyl and the aryl is phenyl.

29. The method according to 27, wherein R is C₁-C₆alkyl, preferably R is methyl, ethyl, n-propyl, isopropyl, n-butyl, sec-butyl, isobutyl, tert-butyl, n-pentyl, sec-pentyl, isopentyl, tert-pentyl, 2,2-dimethylpentyl, hexyl, isohexyl, 2,2-dimethylbutyl, sec-hexyl, or tert-hexyl.

30. The method according to claim 27, wherein x is the same and is an integer from 2 to 5.

31. The method according to claim 27, wherein the titanium (IV) compound is a compound of general formula (I) or (II): 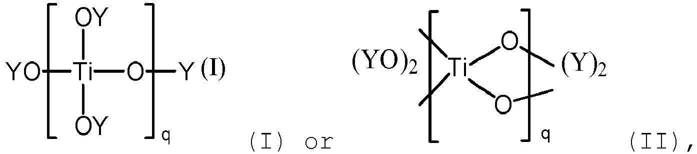 wherein
q is an integer from 1 to 4;
Y independently is R or R'; wherein R or R' is as defined in claim 31,
or a mixture thereof;
with proviso that the compounds of general formula (I) and (II) comprise at least two alkyloxy or two aryloxy groups.

32. The method according to any one of claims 27 to 31, wherein the titanium (IV) compound is a compound selected from:

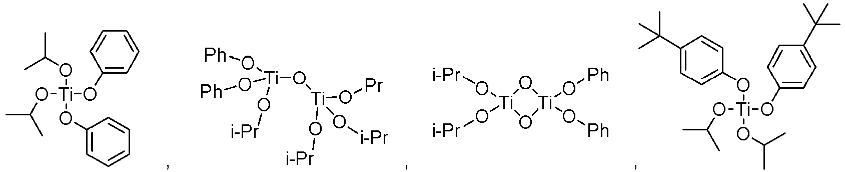

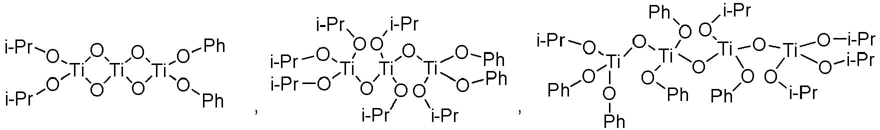

33. The method according to claim 32, wherein the titanium (IV) compound is diisopropoxy-diphenoxy titanium or diisopropoxy-di(p-tretbutyl)phenoxy titanium.

## Patentansprüche

1. Verfahren zur Herstellung eines Esters der Terephthalsäure das die folgenden Schritte umfasst
a) Veresterung von Terephthalsäure mit einem Alkohol der 1 bis 6 Kohlenstoffatome enthält, während kontinuierlich ein Überschuss des Alkohols in dem System aufrechterhalten wird, wobei der Veresterungsschritt zwei aufeinanderfolgende Schritte umfasst: der erste Schritt ist Veresterung unter Verwendung eines metallhaltigen Katalysators; und der zweite Stufe ist die Veresterung unter Verwendung eines sauren Katalysators, um einen rohen Ester;
b) Neutralisierung des Säurekatalysators im Rohester;
c) Entfernung von niedrig siedenden Verunreinigungen aus dem Rohester;
d) Filtration und Trocknung des Esters zur Gewinnung des Esters der Terephthalsäure zu erhalten.

2. Verfahren nach Anspruch 1,
wobei die Veresterung in einem einzigen Reaktionsgefäß durchgeführt wird, bei dem es sich um einen beliebigen Reaktor Reaktor ist, der mit einem Heizelement ausgestattet ist.

3. Verfahren nach Anspruch 1,
wobei der Alkohol, der 1 bis 6 Kohlenstoffatome enthält, ein einatomiger Alkohol ist, der vorzugsweise 2 bis 5 Kohlenstoffatome, am meisten bevorzugt 3 bis 4 Kohlenstoffatome Atomen, am meisten bevorzugt Butanol.

4. Verfahren nach Anspruch 1,
wobei ein Verhältnis von Terephthalsäure: Alkohol von 1:1 bis 1:3, vorzugsweise von 1:2 bis 1:3, am meisten bevorzugt 1:2,5 bis 1:2,7.

5. Verfahren nach Anspruch 1,
wobei der metallhaltige Katalysator, der in der ersten Stufe zugesetzt wird, eine Verbindung von Titan, Zinn oder Zirkonium, vorzugsweise eine Titan(IV)-Verbindung, besonders bevorzugt Titantetraisopropoxid, Titantetraisobutoxid Tetraisobutoxid, oder eine Zusammensetzung, die durch eine Reaktion von Titanorthoester mit einem Alkohol, einer Säure und/oder einer Base hergestellt wird.

6. Verfahren nach Anspruch 1,
wobei eine Menge des metallhaltigen Katalysators von 0,1 bis 1 Gew.-%, vorzugsweise von 0,5 bis 0,6 Gew.-% beträgt.

7. Verfahren nach Anspruch 1,
wobei der Säurekatalysator eine Verbindung ist, die ausgewählt ist aus der Gruppe bestehend aus
Schwefelsäure, Methansulfonsäure, vorzugsweise para-Toluolsulfonsäure.

8. Verfahren nach Anspruch 1,
wobei eine Menge des Säurekatalysators von 0,1 bis 0,5 Gew.-%, vorzugsweise von 0,2 bis 0,3 Gew.-% ist.

9. Verfahren nach Anspruch 1,
wobei der Säurekatalysator in der zweiten Stufe 2,5 bis 5,5 Stunden, vorzugsweise 3,0 bis 4,0 Stunden, nach Beginn der Veresterungsstufe, d.h., nach dem Beginn des Erhitzens der Terephthalsäure und des Alkohol.

10. Verfahren nach Anspruch 1,
wobei die Temperatur der Veresterungstemperatur in der ersten Stufe 160 bis einschließlich 185°C und 185 bis einschließlich 220°C in der zweiten Stufe.

11. Verfahren nach Anspruch 1,
wobei die Dauer des der Veresterungsstufe 3,5 bis 9 Stunden, vorzugsweise 4 bis 7 Stunden, am meisten bevorzugt 5 bis 6 Stunden.

12. Verfahren nach Anspruch 1,
wobei der Druck bei der Veresterungsstufe 4 bis 7 atm. (4-7 bar), vorzugsweise von 5 bis 6 atm. (5-6 bar), am meisten bevorzugt 6 atm. (6 bar).

13. Verfahren nach Anspruch 1,
wobei ein Adsorptionsmittel verwendet wird.

14. Verfahren nach Anspruch 13,
wobei das Adsorptionsmittel Aktivkohle, Zeolith oder Perlit ist.

15. Verfahren nach Anspruch 13,
wobei die Menge des verwendeten Adsorptionsmittels von 0,01 bis 2 Gew.-%, vorzugsweise von 0,1 bis 1 Gew.-%.

16. Verfahren nach Anspruch 1,
wobei der Schritt der Veresterung die Entfernung eines azeotropen Alkohol-WasserGemisches Gemisches umfasst.

17. Verfahren nach Anspruch 1,
wobei ein Überschuss an Alkohol durch Abtrennung eines Alkohol-Wasser-Azeotrop-Gemisches aufrechterhalten wird und anschließender Rückführung des Alkohols in den Veresterungsschritt.

18. Verfahren nach Anspruch 1,
bei dem ein Überschuss an Alkohol aufrechterhalten wird, indem wasserfreier Alkohol aus einem separaten Behälter zugefügt wird.

19. Verfahren nach Anspruch 1,
**dadurch gekennzeichnet, dass**
bei dem Neutralisierungsschritt ein Neutralisierungsmittel verwendet wird, das eine Lösung von Natriumhydroxid; Natrium, Kalium oder Magnesiumcarbonat, Natriumsilikat, Kaliumhydroxid oder dergleichen ist.

20. Verfahren nach Anspruch 19,
wobei eine Menge von des Neutralisationsmittels 0,5 bis 6 Mol pro Mol nicht umgesetzter Säure, vorzugsweise von 3 bis 4 Mol pro Mol nicht umgesetzter Säure, beträgt.

21. Verfahren nach Anspruch 1,
wobei der Schritt der Neutralisierung bei einer Temperatur von 50 bis einschließlich 90°C durchgeführt wird.

22. Verfahren nach Anspruch 1,
bei dem niedrig siedende Verunreinigungen in einer Säulenapparatur entfernt werden.

23. Verfahren nach Anspruch 22,
wobei die Vorrichtung in einem Blasenmodus arbeitet.

24. Verfahren nach Anspruch 1,
wobei der Schritt der Esterfiltration bei einer Temperatur von 50 bis 90°C durchgeführt wird.

25. Verfahren nach Anspruch 1,
wobei der Schritt der Trocknung bei einer Temperatur von 105 bis einschließlich 115°C durchgeführt wird.

26. verfahren nach Anspruch 1,
bei dem der Schritt der Trocknung bei einem Druck von 0,05 bis 0,1 atm durchgeführt wird (0,05 bis 0,1 bar).

27. Verfahren nach Anspruch 1,
umfassend die Schritte:
a) Veresterung von Terephthalsäure mit einem Alkohol der 1 bis 6 Kohlenstoffatome umfasst, während kontinuierlich ein Überschuss des Alkohols in dem System, wobei die Veresterung zwei aufeinanderfolgende Schritte umfasst:
der erste Schritt ist die Veresterung unter Verwendung einer metallhaltigen Katalysator eine Titan(IV)-Verbindung der allgemeinen Formel:
Zinn(OR)x(OR')xOy
worin
n eine ganze Zahl von 1 bis 4 ist;
y eine ganze Zahl von 0 bis 6 ist;
x gleich oder verschieden sein kann und eine ganze Zahl von 2 bis 8 ist;
R ist ein lineares oder verzweigtes C1-C18-Alkyl, C3-C18-Cycloalkyl, R' ist Aryl ist, das gegebenenfalls einen Elektronendonor-Substituenten enthält;
oder ein Gemisch davon,
mit der Maßgabe, daß
wenn n gleich 1 ist, dann ist x gleich 2 und y gleich O; und,
wenn n>l ist, dann umfassen die Verbindungen mindestens zwei Alkoxygruppen und zwei Aryloxygruppen umfassen;
und der zweite Schritt die Veresterung unter Verwendung eines sauren Katalysators, um einen rohen Ester herzustellen;
b) Neutralisierung des Säurekatalysators im Rohester;
c) Entfernung von niedrig siedenden Verunreinigungen aus dem Rohester;
d) Filtration und Trocknung des Esters, um den Ester der Terephthalsäure zu erhalten.

28. Verfahren nach Anspruch 27,
wobei R' Phenyl ist, gegebenenfalls umfassend einen Elektronendonor-Substituenten, vorzugsweise der Elektronendonor-Substituent eine C1-C6-Alkyl-, Aryl-, C1-C6-Alkoxy-, C1-C6-Dialkylamino- oder C1-C6-Alkylthiogruppe ist, am meisten bevorzugt der Alkylteil im Elektronendonor-Substituenten vorzugsweise Methyl ist, Ethyl, n-Propyl, Isopropyl, n-Butyl, sec-Butyl, Isobutyl, tert-Butyl, n-Pentyl, sec-Pentyl, Isopentyl, tert-Pentyl, 2,2 Dimethylpentyl, Hexyl, Isohexyl, 2,2-Dimethylhexyl, sec-Hexyl oder tert-Hexyl und das Aryl ist Phenyl.

29. Verfahren nach 27,
wobei R für C1-C6-Alkyl steht, vorzugsweise ist R Methyl, Ethyl, n-Propyl, Isopropyl, n-Butyl, sec-Butyl, Isobutyl, tert-Butyl, n-Pentyl, sec-Pentyl, Isopentyl, tert-Pentyl, 2,2-Dimethylpentyl, Hexyl, Isohexyl, 2,2 Dimethylbutyl, sec-Hexyl oder tert-Hexyl.

30. Verfahren nach Anspruch 27,
wobei x die gleiche Bedeutung hat ist und eine ganze Zahl von 2 bis 5 ist.

31. Verfahren nach Anspruch 27,
wobei die Titan (IV)-Verbindung eine Verbindung der allgemeinen Formel (I) oder (II) ist: 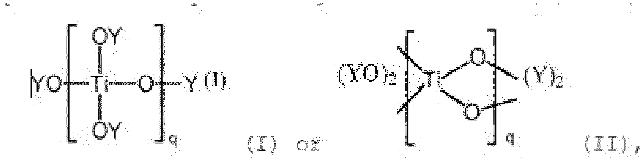 wobei q eine ganze Zahl von 1 bis 4 ist; Y unabhängig voneinander R oder R' ist; wobei R oder R' wie definiert ist in Anspruch 31, oder ein Gemisch davon; mit der Maßgabe, dass die Verbindungen der allgemeinen Formel (I) und (II) mindestens zwei Alkyloxy- oder zwei Aryloxygruppen umfassen.

32. Verfahren nach einem der Ansprüche 27 bis 31,
wobei die Titan(IV)-Verbindung eine Verbindung ist, ausgewählt aus:

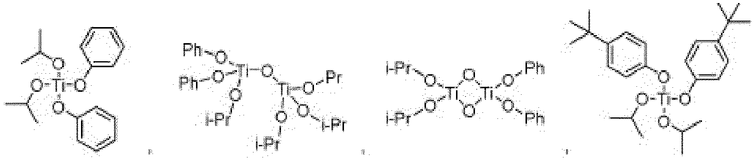

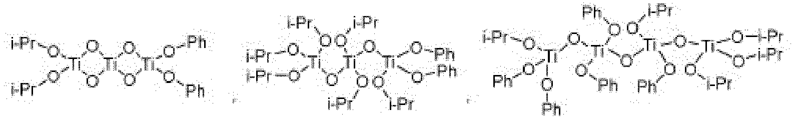

33. Verfahren nach Anspruch 32,
wobei die Titan (IV)-Verbindung Diisopropoxy-Diphenoxy-Titan oder Diisopropoxydi(p-tretbutyl)phenoxy-Titan ist.

## Revendications

1. Méthode de préparation d'un ester de l'acide téréphtalique, comprenant les étapes suivantes
a) estérification de l'acide téréphtalique avec un alcool comprenant de 1 à 6 atomes de carbone, tout en maintenant continuellement un l'alcool dans le système, l'étape d'estérification comprenant deux étapes séquentielles l'étape d'estérification comprend deux étapes séquentielles : la première étape est l'estérification par estérification à l'aide d'un catalyseur contenant un métal ; et
la deuxième étape est l'estérification à l'aide d'un catalyseur acide, pour produire un ester brut ;
b) neutralisation du catalyseur acide dans l'ester brut ;
c) élimination des impuretés à faible point d'ébullition de l'ester brut ;
d) filtration et séchage de l'ester pour obtenir l'ester de l'acide téréphtalique. l'acide téréphtalique.

2. Méthode selon la revendication 1,
dans laquelle l'estérification est effectuée dans une seule cuve de réaction, qui est tout réacteur réacteur connu dans l'art antérieur, équipé d'un élément chauffant.

3. Méthode selon la revendication 1,
dans laquelle l'alcool comprenant 1 à 6 atomes de carbone est un alcool monoatomique comprenant de préférence 2 à 5 atomes de carbone, de préférence 3 à 4 atomes de carbone atomes de carbone, de préférence le butanol.

4. Méthode selon la revendication 1,
dans laquelle le rapport téréphtalique: alcool est de 1:1 à 1:3, de préférence de 1:2 à 1:3, de préférence encore de 1:2,5 à 1:2,7.

5. Méthode selon la revendication 1,
dans laquelle le catalyseur contenant un metal ajouté à la première étape est un composé de titane, d'étain ou de zirconium, de préférence un composé de titane (IV), de préférence le tétraisopropoxyde de titane, le tétraisobutoxyde de titane, ou une composition préparée par une réaction de l'orthoester de titane avec un alcool.

6. Méthode selon la revendication 1,
dans laquelle une quantité de catalyseur contenant des métaux est de 0,1 à 1 % en poids, de préférence de 0,5 à 0,6 % en poids.

7. Méthode selon la revendication 1,
dans lequel le catalyseur acide est un composé choisi dans le groupe constitué par l'acide sulfurique, l'acide méthanesulfonique, l'acide para-toluènesulfonique, de préférence l'acide para-toluènesulfonique.

8. Méthode selon la revendication 1,
dans laquelle la quantité de catalyseur acide est de 0,1 à 0,5 % en poids, de préférence de 0,2 à 0,3 % en poids.

9. Méthode selon la revendication 1,
dans laquelle le catalyseur acide est ajouté à la deuxième étape entre 2,5 et 5,5 heures, de préférence entre 3,0 et 4,0 heures, après le début de l'étape d'estérification, c'est-à-dire après le début du chauffage de l'acide téréphtalique et de l'alcool.

10. Procédé selon la revendication 1,
dans lequel la température d'estérification est comprise entre 160 et 185°C au cours de la première étape, et entre 185 et 220°C au cours de la deuxième étape.

11. Méthode selon la revendication 1,
dans laquelle la durée de l'étape d'estérification est de 3,5 à 9 heures, de préférence de 4 à 7 heures, de préférence encore de 5 à 6 heures.

12. Méthode selon la revendication 1,
dans laquelle la pression à l'étape d'estérification est de 4 à 7 atm (4-7 bar), de préférence de 5 à 6 atm. (5-6 bar), de préférence 6 atm. (6 bar).

13. Méthode selon la revendication 1,
dans laquelle on utilise un adsorbant.

14. Méthode selon la revendication 13,
dans laquelle l'adsorbant est du charbon actif, de la zéolithe ou de la perlite.

15. Méthode selon la revendication 13,
dans laquelle une quantité de l'adsorbant utilisé est de 0,01 à 2 % en poids, de préférence de 0,1 à 1 % en poids.

16. Méthode selon la revendication 1,
dans laquelle l'étape estérification comprend l'élimination d'un mélange azéotropique alcool-eau.

17. Méthode selon la revendication 1,
dans laquelle un excès d'alcool est maintenu par la séparation d'un mélange azéotropique alcool-eau, suivi du recyclage de l'alcool dans l'étape d'estérification.

18. Méthode selon la revendication 1,
dans laquelle un excès d'alcool est maintenu par d'alcool anhydre provenant d'un récipient séparé.

19. Méthode selon la revendication 1,
dans laquelle, lors de l'étape de neutralisation, un agent neutralisant qui est une solution d'hydroxyde de sodium, de carbonate de sodium, de potassium ou de magnésium, de silicate de sodium, d'hydroxyde de potassium ou similaire.

20. Méthode selon la revendication 19,
dans laquelle une quantité de l'agent neutralisant est de 0,5 à 6 moles par mole d'acide non réagi, de préférence de 3 à 4 moles par mole d'acide non réagi.

21. Méthode selon la revendication 1,
dans laquelle l'étape de neutralisation est effectuée à une température comprise entre 50 et 90°C inclus.

22. Méthode selon la revendication 1,
dans laquelle les impuretés à faible point d'ébullition sont éliminées dans un appareil à colonne.

23. Méthode selon la revendication 22,
dans laquelle l'appareil fonctionne en mode bulle.

24. Méthode selon la revendication 1,
dans lequel l'étape de la filtration de l'ester est effectuée à une température de 50 à 90°C.

25. Procédé selon la revendication 1,
dans lequel l'étape de séchage est effectuée à une température comprise entre 105 et 115°C inclusivement.

26. méthode selon la revendication 1,
dans laquelle l'étape de séchage est effectuée sous une pression de 0,05 à 0,1 atm (0,05 à 0,1 bar).

27. Méthode selon la revendication 1,
comprenant les étapes suivantes
a) estérification de l'acide téréphtalique avec un alcool comprenant de 1 à 6 atomes de carbone, tout en maintenant continuellement un l'alcool dans le système, l'estérification comprenant deux étapes séquentielles:
la première étape est l'estérification en utilisant comme catalyseur contenant un métal, un composé de titane (IV) de formule générale :
Étain(OR)x(OR')xOy
dans lequel
n est un nombre entier de 1 à 4 ;
y est un nombre entier de O à 6 ;
x peut être identique ou différent et est un nombre entier de 2 à 8 ;
R est un alkyle en C1-C18 linéaire ou ramifié, un cycloalkyle en C3-C18, R' est un aryle comprenant éventuellement un électron-don.
un aryle comprenant éventuellement un substituant donneur d'électrons ;
ou un mélange de ceux-ci,
sous réserve que
si n est égal à 1, x est égal à 2 et y est égal à O ; et,
si n>l, les composés comprennent au moins deux groupes alcoxy et deux groupes aryloxy ;
et la deuxième étape est l'estérification à l'aide d'un catalyseur acide, afin de produire un ester brut;
b) neutralisation du catalyseur acide dans l'ester brut ;
c) élimination des impuretés à faible point d'ébullition de l'ester brut ;
d) filtration et séchage de l'ester pour obtenir l'ester de l'acide téréphtalique.

28. Méthode selon la revendication 27,
dans lequel R' est un phényle comprenant éventuellement un substituant donneur d'électrons, de préférence le substituant donneur d'électrons est un groupe alkyle en C1-C6, aryle, alcoxy en C1-C6, C1 -C6dialkylamino, ou un groupe C1 -C6alkyl thio, de préférence la partie alkyle dans le groupe donneur d'électrons est un groupe alkyle. la partie alkyle du substituant donneur d'électrons est de préférence un groupe méthyle, éthyle, n-propyle, isopropyle, n-butyle, sec-butyle, isobutyle, tertbutyle, n-pentyle, sec-pentyle, isopentyle, tert-pentyle, 2,2- diméthylpentyle, hexyle, isohexyle, 2,2-diméthylhexyle, sec-hexyle ou tert-hexyle et l'aryle est le phényle.

29. Méthode selon 27,
dans laquelle R est un alkyle en C1-C6, de préférence R est un méthyle, un éthyle, un n-propyle, un isopropyle, un n-butyle, un secbutyle, un isobutyle, un tert-butyle, isobutyle, tert-butyle, n-pentyle, sec-pentyle, isopentyle, tert-pentyle, 2,2-diméthylpentyle, hexyle, isohexyle, 2,2- diméthylbutyle, sec-hexyle ou tert-hexyle.

30. Méthode selon la revendication 27,
dans laquelle x est le même et est un nombre entier compris entre 2 et 5.

31. Méthode selon la revendication 27,
dans laquelle le composé de titane (IV) est un composé de formule générale (I) ou (II) : 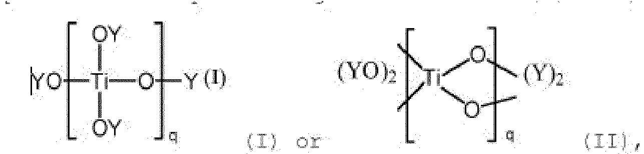 dans lequel q est un nombre entier de 1 à 4 ; Y est indépendamment R ou R' ; dans lequel R ou R' est tel que défini dans la revendication 31, ou un mélange de ceux-ci ; à condition que les composés de formule générale (I) et (II) (II) comprennent au moins deux groupes alkyloxy ou deux groupes aryloxy.

32. Méthode selon l'une des revendications 27 à 31,
dans laquelle le composé de titane (IV) est un composé choisi parmi :

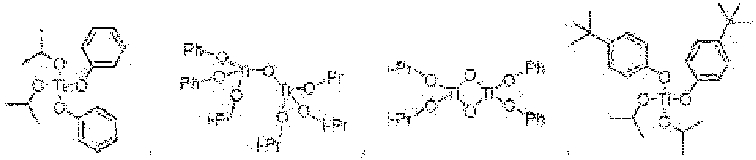

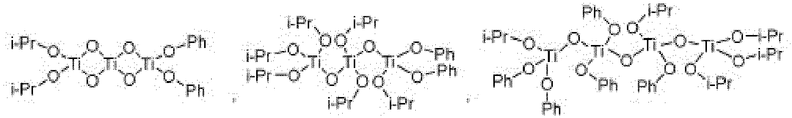

33. Méthode selon la revendication 32,
dans laquelle le composé de titane (IV) est le diisopropoxy-diphénoxy titane ou le diisopropoxydi(p-tretbutyl)phénoxy titane.
